# EUROPÄISCHE PATENTSCHRIFT

(11) **EP 1 276 749 B1**
(45) Veröffentlichungstag und Bekanntmachung des Hinweises auf die Patenterteilung: **24.08.2005**
(21) Anmeldenummer: 01929464.4
(22) Anmeldetag: 26.03.2001
(51) Int. Cl.: C07H 19/20, A61K 31/7076, A61K 31/708

(54) **PHOTOLABILE CUMARINYLMETHYLESTER VON CYCLISCHEN NUCLEOTIDEN, VERFAHREN ZU DEREN HERSTELLUNG UND IHRE VERWENDUNG**
PHOTO-LABILE COUMARINYLMETHYL ESTERS OF CYCLIC NUCLEOTIDES, METHOD FOR THE PRODUCTION AND USE THEREOF
COUMARINYLE METHYLESTERS PHOTOLABILES DE NUCLEOTIDES CYCLIQUES, PROCEDE DE FABRICATION ET UTILISATION DE CEUX-CI

(30) Priorität: 20.04.2000 DE 10021256
(43) Veröffentlichungstag der Anmeldung: 22.01.2003
(73) Patentinhaber: Forschungsverbund Berlin e.V., 12489 Berlin (DE); FORSCHUNGSZENTRUM JÜLICH GMBH, 52425 Jülich (DE)
(72) Erfinder: HAGEN, Volker, 13051 Berlin (DE); KAUPP, Ulrich, Benjamin, 52062 Aachen (DE); BENDIG, Jürgen, 10369 Berlin (DE); WIESNER, Burghard, 12621 Berlin (DE)
(74) Vertreter: Ziebig, Marlene
(86) Internationale Anmeldenummer: PCT/EP2001/003512
(87) Internationale Veröffentlichungsnummer: WO 2001/081360

(56) Entgegenhaltungen:
- B. SCHADE ET AL.: "Deactivation and excited-state properties of (coumarin-4-yl)methyl derivatives. 1. Photocleavage of (7-methoxycoumarin-4-yl)methyl-caged acids with fluorescence enhancement" J. ORG. CHEM., Bd. 64, 1999, Seiten 9109-9117, XP002170184 in der Anmeldung erwähnt
- V. HAGEN ET AL.: "Highly efficient and ultrafast phototriggers for cAMP and cGMP by using long-wavelength UV/Vis-activation" ANGEW. CHEM. INT. ED., Bd. 40, 2001, Seiten 1046-1048, XP002170185

## Beschreibung

Die Erfindung betrifft photolabile Cumarinylmethylester von cyclischen Nucleotiden der allgemeinen Formel I 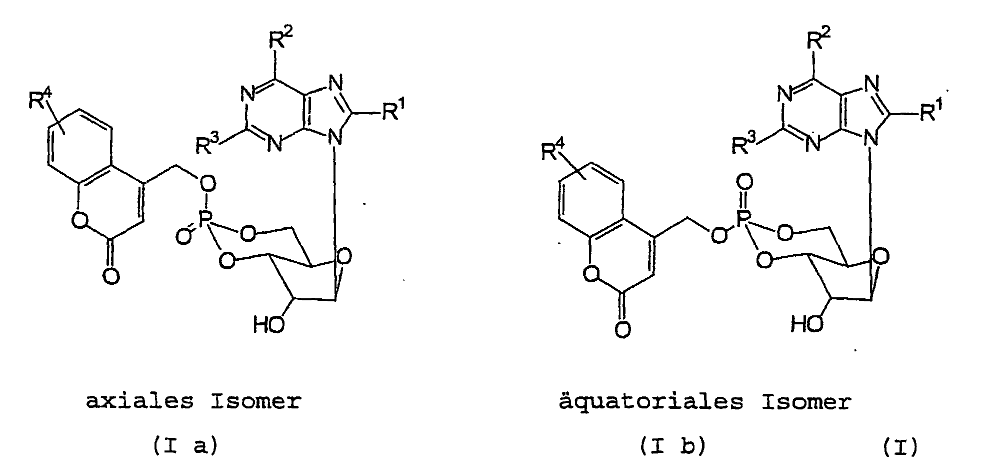 in der
- R¹: Wasserstoff, Brom oder p-Chlorphenylthio bedeutet,
- R⁴: 7-Carboxymethoxy, 6,7-, 5,7- oder 7,8- Bis(carboxymethoxy), 7-C₁-C₃-Alkoxycarbonylmethoxy, 6,7-Bis (C₁-C₃-alkoxycarbonylmethoxy), 5,7-Bis(C₁-C₃-alkoxycarbonylmethoxy) oder 7,8-Bis(C₁-C₃-alkoxycarbonylmethoxy) darstellt,
- R²: -NH₂ bedeutet und R³ Wasserstoff ist (Adeninrest) oder
- R²: -OH bedeutet und R³ -NH₂ darstellt (Guaninrest).

Ferner betrifft die Erfindung ein Verfahren zur Herstellung der Verbindungen der Formel I sowie deren Verwendung als biologisch inaktive, photolabile Precursoren zur photochemischen Freisetzung der entsprechenden biologisch aktiven cyclischen Nucleotide zur Untersuchung cyclisch Nucleotid-abhängiger zellulärer Prozesse in biologischen Systemen.

Es ist bekannt, daß Nucleotide wie cyclisches Guanosin-3',5'-monophosphat (cGMP) und cyclisches Adenosin-3',5'-monophosphat (cAMP) eine Vielzahl wichtiger zellulärer Prozesse kontrollieren. Um solche cyclisch Nucleotid-abhängigen Prozesse zu beeinflussen und zeitlich und mechanistisch zu untersuchen, werden chemische Derivate dieser Nucleotide (sog. "caged" Verbindungen), die biologisch inaktiv und photolabil sein müssen, in biologische Systeme eingebracht und durch Bestrahlung mit Licht in die biologisch aktiven cyclischen Nucleotide überführt. Als biologische Systeme werden bei diesen Untersuchungen in der Regel Zelllinien verwendet.

Dabei werden an die eingesetzten caged Verbindungen hohe Anforderungen gestellt. Sie sollen ausreichend hohe Löslichkeiten in wäßrigen Pufferlösungen, schnelle und mit hoher Effizienz verlaufende photochemische Reaktionen, hohe Extinktionen bei Wellenlängen > 300 nm, möglichst geringe Hydrolyseempfindlichkeiten, geringe Toxizitäten und keine relevanten biologischen Wirkungen zeigen. Bisher sind zwar in der Literatur eine ganze Reihe photolabiler cyclischer Nucleotidester beschrieben worden, aber alle weisen eine Reihe von Nachteilen auf. Auch liegen gegenwärtig noch nicht genügend Daten vor, um a priori die Eigenschaften neuer caged Verbindungen vorherzusagen und stets ist zusätzlich mit einer unerwünschten präphotolytischen biologischen Aktivität bei der Anwendung auf zelluläre Systeme zu rechnen [K. R. Gee et. al., Biochemistry 38, 3140 (1999)].

Bisher wurden die 2-Nitrobenzyl-, 4,5-Dimethoxy-2-nitrobenzyl- und 1-(2-Nitrophenyl)ethylester von cyclischem Guanosin-3',5'-monophosphat (cGMP) und cyclischem Adenosin-3',5'-monophosphat (cAMP) sowie der (7-Methoxy-cumarin-4-yl)methylester von cAMP [T. Furuta et al., J. Org. Chemistry 60, 3953 (1995)], der 7-Hydroxy- und verschiedene Acyloxycumarinylmethylester von cAMP [T. Furuta et al. Methods in Enzymology, Vol. 291 (Ed. G. Marriott), 50 (1998), T. Furuta et. al., Biochem. Biophys. Res. Commun. 228, 193 (1996)] in der Literatur beschrieben. Eingesetzt wurden auch die 4,5-Dimethoxy-2-nitrobenzyl-, 1-(2-Nitrophenyl)ethyl- und (7-Methoxy-cumarin-4-yl)methyl-ester von 8-Br- bzw. 8-p-Chlorphenylthio-cAMP und 8-Br- bzw. 8-p-Chlorphenylthio-cGMP [WO 97/05155; V. Hagen et. al., Biochemistry, 35, 7762 (1996); V. Hagen et. al., J. Photochem. Photobiol. B: Biol. 42, 71 (1998); V. Hagen et. al., J. Photochem. Photobiol. B: Biol. 53, 91(1999] und der 4,5-Bis(carboxymethoxy)-2-nitrobenzylester von cGMP [V. Lev-Ram et al., Neuron 18, 1025 (1997)].

Beschrieben, aber bisher offenbar nicht als caged Verbindungen in biologischen Experimenten eingesetzt, wurden auch der (7-Methoxycumarin-4-yl)methylester von cGMP [B. Schade et. al., J. Org. Chem. 64, 9109 (1999)], der Desylester von cAMP [R. S. Givens et. al., J. Am. Chem. Soc. 114, 8708 (1992)] der (Anthrachinon-2-yl)methyl- und der (Naphth-2-yl)methylester von cAMP [T. Furuta et. al., J. Org. Chem. 60, 3953 (1995)].

Alle bisher beschriebenen photolabilen cyclischen Nucleotidester weisen eine Reihe von Nachteilen auf.

In der Regel ist die Löslichkeit in wäßrigen Pufferlösungen für die beschriebenen caged cyclischen Nucleotide, bedingt durch die Veresterung der freien Phosphorsäuregruppierung, eingeschränkt.

Darüber hinaus zeigen die 4,5-Dimethoxy-2-nitrobenzylester der cyclischen Nucleotide cAMP und cGMP und ihrer Derivate relativ niedrige Quantenausbeuten bei der Photolyse, eine geringe Photolysegeschwindigkeit und außerdem sind insbesondere die äquatorialen Isomeren in wäßrigen Pufferlösungen solvolyseempfindlich [V. Hagen et. al., Biochemistry, 35, 7762 (1996)]. Vom 4,5-Bis(carboxymethoxy)-2-nitroben-zylester von cGMP sind bisher keine phys.-chemischen Daten bekannt. Aus dem mit den vorstehenden Verbindungen gemeinsamen Photolyse- bzw. Solvolysemechanismus ist anzunehmen, daß dieser Ester analoge nachteilige Eigenschaften aufweisen sollte.

Nachteilig bei den bisher genutzten 1-(2-Nitrophenyl)ethylestern der cyclischen Nucleotide sind die langsame Freisetzungskinetik und die geringe Absorption bei Wellenlängen größer 300 nm. Eine geringe Absorption führt - ebenso wie eine geringe Quantenausbeute der Photolyse - zu einer unbefriedigenden Effizienz der Photolyse, was zwar durch energiereichere Bestrahlung oder längere Belichtungszeiten kompensiert werden kann, aber in der Regel zu Zellschädigungen führt.

Der Desylester von cAMP ist in wäßrigen Pufferlösungen solvolyseempfindlich und der (Anthrachinon-2-yl)methyl- und der (Naphth-2-yl)methylester von cAMP sind in wäßrigen Pufferlösungen zu wenig löslich, so daß diese Verbindungen für viele Fragestellungen als Phototrigger für cAMP ungeeignet sind. Auch die Anwendung der (7-Methoxy-cumarin-4-yl)-methylester von cAMP und cGMP bzw. von 8-Br-cAMP oder 8-Br-cGMP ist durch die relativ geringe Löslichkeit in wäßrigen Pufferlösungen stark eingeschränkt. Zudem wurde für das äquatoriale Isomere von dem (7-Methoxy-cumain-4-yl)methylester von cAMP eine relativ niedrige hydrolytische Halbwertszeit (60 h) in Ringer's Lösung beschrieben.

Für die 7-Hydroxy- und 7-Acyloxycumarinylmethylester von cAMP wurden gleichfalls niedrige hydrolytische Halbwertszeiten beschrieben und die photochemischen Quantenausbeuten sind nur halb so hoch wie die des 7-Methoxycumarinylmethylesters. Außerdem dürfte die Löslichkeit der Verbindungen eingeschränkt sein.

Aufgabe der vorliegenden Erfindung war es, neue, photolabile cyclische Nucleotidderivate bereitzustellen, die bei hoher Solvolysebeständigkeit sowohl eine hohe Löslichkeit in wäßrigen Pufferlösungen aufweisen sollen als auch eine hohe Effizienz der Photospaltung und eine möglichst schnelle Freisetzungskinetik zeigen sollen. Dabei ist auch von Wichtigkeit, daß die Verbindungen keine präphotolytische biologische Aktivität aufweisen, um sie zur Untersuchung zellulärer Systeme anwenden zu können.

Die Aufgabe der Erfindung wird durch neue Cumarinylmethylester von cyclischem Guanosin-3',5'-monophosphat (cGMP) und cyclischem Adenosin-3',5'monophosphat (cAMP) gemäß der allgemeinen Formel I, 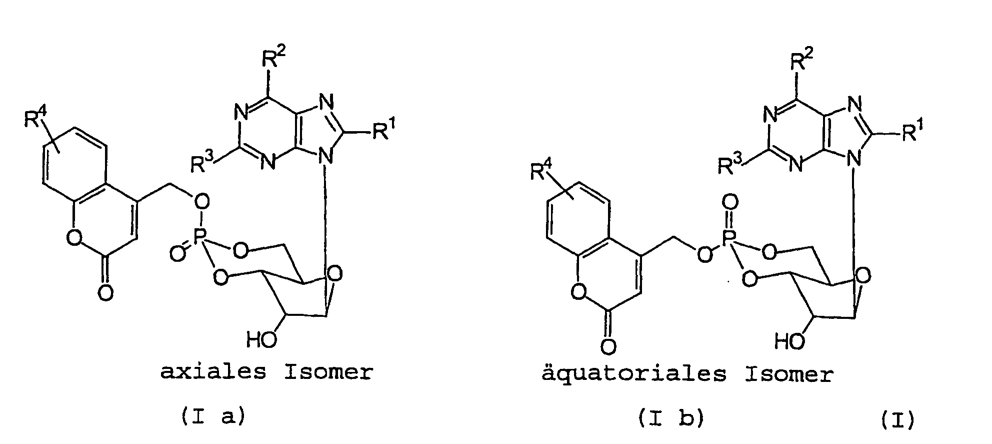 in der
- R¹: Wasserstoff, Brom oder p-Chlorphenylthio bedeutet,
- R⁴: 7-Carboxymethoxy, 6,7-, 5,7 oder 7,8- His(carboxymethoxy), 7-C₁-C₃-Alkoxycarbonylmethoxy, 6,7-Bis (C₁-C₃-alkoxycarbonylmethoxy) 5,7-Bis (C₁-C₃-alkoxycarbonylmethoxy) oder 7,8-Bis(C₁-C₃-alkoxycarbonylmethoxy) darstellt,
- R²: -NH₂ bedeutet und R³ Wasserstoff ist (Adeninrest) oder
- R²: -OH bedeutet und R³ -NH₂ darstellt (Guaninrest),
gelöst, wobei die Verbindungen mit dem Guaninrest (cGMP) in Dihydropyrimidonstruktur vorliegen.

Bevorzugte Verbindungen sind solche mit R⁴ als Carboxymethoxy oder Bis(carboxymethoxy). Ganz besonders bevorzugt sind die 6,7-Bis(carboxymethoxy)- oder 6,7-Bis(C₁-C₃-alkoxycarbonylmethoxy)-substituierten Verbindungen.

In einer bevorzugten Ausführungsform der Erfindung bedeutet C₁-C₃ -Alkoxy im Rest R⁴ den Methoxy- oder Ethoxyrest.

Im Sinne der Erfindung besonders bevorzugte Verbindungen sind die folgenden Verbindungen 1 - 11:
1. [6,7-Bis(carboxymethoxy)cumarin-4-yl]methylester von cAMP
2. [7,8-Bis(carboxymethoxy)cumarin-4-yl]methylester von cAMP
3. [7-(Carboxymethoxy-cumarin-4-yl]methylester von cGMP
4. [5,7-Bis(carboxymethoxy)cumarin-4-yl]methylester von cGMP
5. [6,7-Bis(carboxymethoxy)cumarin-4-yl]methylester von 8-Br-cAMP
6. [6,7-Bis(carboxymethoxy)cumarin-4-yl]methylester von 8-Br-cGMP
7. [5,7-Bis(carboxymethoxy)cumarin-4-yl]methylester von 8-p-Chlorphenylthio-cAMP
8. [6,7-Bis(methoxycarbonylmethoxy)cumarin-4-yl]methylester von cAMP
9. [6,7-Bis(ethoxycarbonylmethoxy)cumarin-4-yl]methylester von cGMP
10. {6,7-Bis(carboxymethoxy)cumarin-4-yl]methylester von cGMP
11. [7-(Carboxymethoxy-cumarin-4-yl]methylester von cAMP

Erfindungsgemäß ganz besonders bevorzugt sind die äquatorialen Isomeren der allgemeinen Formel I b, insbesondere die mit R⁴ in der Bedeutung von Carboxymethoxy bzw. Bis(carboxymethoxy).

Überraschenderweise zeigen die erfindungsgemäßen Verbindungen ein Eigenschaftsprofil, mit dem sie den bisher beschriebenen caged cyclischen Nucleotiden des Standes der Technik bei der Untersuchung cyclisch Nucleotid-abhäniger zellulärer Prozesse weit überlegen sind. Alle erfindungsgemäßen Cumarinylmethylester sind in wäßrigen Pufferlösungen äußerst hydrolysestabil und die Derivate mit freien Carb-oxymethoxygruppierungen am Cumaringerüst zeigen in wäßrigen Pufferlösungen eine unerwartet hohe Löslichkeit, die das Zehn- bis Fünfundzwanzigfache vergleichbarer Verbindungen des Standes der Technik beträgt und teilweise > 1.000 µmol ist.

Die gegenüber cAMP- und cGMP-Bindungsstellen inaktiven erfindungsgemäßen Verbindungen sind photolabil und lassen sich in wäßrigen Pufferlösungen oder nach Einführung in ein biologisches System durch Bestrahlung mit UV-Licht in die freien, biologisch wirksamen nativen oder 8-substituierten cAMP-Derivate und die freien, biologisch wirksamen nativen oder 8-substituierten cGMP-Abkömmlinge überführen. Als weiteres Photolyseprodukt entstehen die entsprechend substituierten 4-Hydroxymethyl-cumarine. Die Effizienz der Photofreisetzung ist, bedingt durch die relativ hohe Absorption >300 nm und die vergleichsweise hohe Quantenausbeute, gut. Besonders vorteilhaft ist zusätzlich die Rotverschiebung des Absorptionsmaximums bei den 6,7-disubstituierten Verbindungen um etwa 20 nm, was eine effiziente Photolyse bis 380 nm ermöglicht. Überraschenderweise entstehen die freien cyclischen Nucleotide im Nanosekundenbereich, also außerordentlich schnell.

Die erfindungsgemäßen Verbindungen zeigen keinerlei präphotolytische biologische Aktivität und lassen sich sowohl für Untersuchungen in zellfreien Präparationen als auch in chemisch oder mechanisch enthäuteten Zellen bzw. in intakten Zellen, z.B. unter Nutzung der patch-clamp-Technik, vorteilhaft nutzen.

Die erfindungsgemäßen alkoxycarbonylmethoxy-substituierten Verbindungen zeigen eine gute Membrangängikeit und können so durch einfache Inkubation in die Zellen eingeführt werden. Durch in der Zelle vorhandene Esterasen werden intrazellulär die gut wasserlöslichen photolabilen erfindungsgemäß carboxymethoxy-substituierten Cumarinylmethylester der cyclischen Nucleotide freigesetzt. Infolge der durch die Carboxylatgruppen erschwerten Membrangängigkeit wird die Rückresorption eingeschränkt und es können hohe intrazelluläre Konzentrationen der caged Verbindungen erhalten werden.

Bedingt durch die geringe Fluoreszenz der erfindungsgemäßen Cumarinylmethylester im Vergleich zu der hohen Fluoreszenz der bei der Photolyse gebildeten substituierten 4-Hydroxymethyl-cumarine kommt es im Verlauf der Photolyse zu einem sehr starken Fluoreszenzanstieg, der mit dem Photolysegrad korreliert und zum Monitoring bzw. zur Quantifizierung der Menge des freigesetzten cyclischen Nucleotides genutzt werden kann.

Die neuen photolabilen cAMP- und cGMP-ester können für die Untersuchung cyclisch Nucleotid-gesteuerter Vorgänge erfolgreich eingesetzt werden. Beispielsweise bewirkt im Vergleich zu dem 4,5-Dimethoxy-2-nitrobenzylester von cGMP eine wesentlich geringere Lichteinstrahlung bei 333nm von dem [6,7-Bis(carboxymethoxy)cumarin-4-yl]methylester von cGMP eine vergleichbare Aktivierung des olfaktorischen cGMP-abhängigen Kationenkanals und während selbst eine gesättigte Lösung von 7-Methoxy-cumarinylmethyl-caged cGMP in HEPES-Puffer nach Photolyse den olfaktorischen cGMP-abhängigen Kationenkanal nur teilweise aktiviert, wird mit der gut wasserlöslichen Verbindung von Beispiel 3 leicht eine vollständige Aktivierung erreicht.

Gegenstand der Erfindung ist deshalb auch die Verwendung der erfindungsgemäßen Verbindungen zur Untersuchung cyclisch Nucleotid-abhängiger zellulärer Prozesse in biologischen Systemen, indem z. B. der cyclisch Nucleotidgesteuerte Calciumeinstrom in die Zellen gemessen wird.

Andererseits kann der durch Photolyse der erfindungsgemäßen Verbindungen erhaltene Fluoreszenzanstieg zum Monitoring oder zur Quantifizierung der Menge des freigesetzten biologisch aktiven cyclischen Nucleotides cAMP, cGMP, 8-Br-cAMP, 8-Br-cGMP, 8-p-Chlorphenylthio-cAMP oder 8-p-Chlorphenylthio-cGMP genutzt werden. Bedingt durch die geringe Fluoreszenz der Cumarinylmethylester der Formel I im Vergleich zu der hohen Fluoreszenz der bei der Photolyse gebildeten 4-Hydroxymethyl-cumarine kommt es im Verlauf der Photolyse zu einem sehr starken Fluoreszenzanstieg, der mit dem Photolysegrad korreliert.

Die erfindungsgemäßen Verbindungen können sowohl als Isomerengemische oder auch in Form ihrer reinen axialen oder äquatorialen Isomeren eingesetzt werden.

Die erfindungsgemäßen Verbindungen werden hergestellt durch Umsetzen der freien Säuren von cAMP, cGMP, 8-Brom-cAMP, 8-Br-cGMP, 8-p-Chlorphenylthio-cAMP oder 8-p-Chlorphenylthio-cGMP mit den entsprechenden mono- bzw. bis-(niedrigalkoxycarbonylmethoxy)- oder (tert.-butoxycarbonyl-methoxy)-substituierten (Cumarin-4-yl)diazomethanen, wobei in letzterem Falle die dabei gebildeten tert.-butoxycarbonylmethoxy-substituierten Cumarinylmethylester der cyclischen Nucleotide, gegebenenfalls nach Isomerentrennung, mit Trifluoressigsäure in die entsprechenden carboxymethoxy-substituierten Cumarinylmethylester der cyclischen Nucleotide überführt werden.

Die im Ergebnis der Alkylierungsreaktion anfallenden substituierten Cumarinylmethylester der cyclischen Nucleotide fallen als Isomerengemisch aus axialer und äquatorialer Form an. Die Aufreinigung der Rohprodukte und gegebenenfalls die Trennung der Isomerengemische wird vorzugsweise chromatografisch durchgeführt. Hierbei hat sich als besonders vorteilhaft der Einsatz von flashChromatographie und/oder HPLC erwiesen.

### Ausführungsbeispiele

### Beispiel 1

### [6,7-Bis(carboxymethoxy)cumaria-4-yl]methylester von cAMP

Eine Mischung aus 1 mmol cAMP und 1,5 mmol [6,7-Bis(tert.-butoxycarbonylmethoxy)cumarin-4-yl]diazomethan (hergestellt aus käuflichem 6,7-Dihydroxy-4-methylcumarin durch Alkylierung mit Bromessigsäure-tert.-butylester, anschließende Überführung in den 6,7-Bis(tert.-butoxycarbonylmethoxy)cumarin 4-carbaldehyd durch Oxidation mit Selendioxid, Reaktion des Aldehyds mit p-Toluensulfonylhydrazin zum entsprechenden p-Toluensulfonylhydrazon und nachfolgende Umwandlung in die Diazoverbindung durch Behandlung mit Triethylamin) in 8 ml DMSO und 32 ml Acetonitril wird unter Lichtausschluß 24 Stunden bei 60°C gerührt. Acetonitril wird abrotiert und das DMSO und einige Nebenprodukte werden nach Abkühlen auf Raumtemperatur durch mehrmaliges Ausschütteln mit Ether entfernt. Der Rückstand, der den [6,7-Bis(tert.-butoxycarbonylmethoxy)cumarin-4-yl]methylester von cAMP enthält, wird mittels flash Chromatographie (Elution mit Chloroform/Methanol) und/oder präparative HPLC (PLRP-S, 10 µm 250 × 25 mm i.d., Flußgeschwindigkeit 10 ml/Min., linearer Gradient 20%-60% B in 70 Min., Eluent A: Wasser, Eluent B: Acetonitril) gereinigt und gegebenenfalls gleichzeitig in das axiale und das äquatoriale Isomer aufgetrennt.
20 Min. Rühren der reinen axialen bzw. der reinen äquatorialen Form oder eines Gemisches aus beiden Formen des [6,7-Bis(tert.-butoxycarbonylmethoxy)cumarin-4-yl]methyl-esters von cAMP in Chloroform/TFA/Wasser (25:74:1; ca. 2,5 ml pro 20 mg) liefert nach Entfernen der Lösungsmittel und Lyophilisierung den [6,7-His(carboxymethoxy)cumarin-4-yl]-methylester von cAMP als reines axiales oder reines äquatoriales Isomer bzw. als Isomerengemisch.
Gesamtausbeute 25 % d. Th.
- **axiales Isomer:**: ³¹P-NMR (DMSO-d₆): δ -5,03;
UV: λₘₐₓ (ε) : 346 (12000)
- **äquatoriales Isomer:**: ³¹P-NMR (DMSO-d₆) : δ -3,26;
UV: λₘₐₓ (ε) : 347 (12000)

### Beispiel 2

### [7,8-Bis(carboxymethoxy)cumarin-4-yl]methylester von cAMP

Analog Beispiel 1 aus 1 mmol cAMP und 1,5 mmol [7,8-Bis(tert.-butoxycarbonylmethoxy)cumarin-4-yl]diazomethan (hergestellt aus käuflichem 7,8-Dihydroxy-4-methylcumarin durch Alkylierung mit Bromessigsäure-tert.-butylester, anschließende Überführung in den 7,8-Bis(tert.-butoxycarbonylmethoxy)cumarin 4-carbaldehyd durch Oxidation mit Selendioxid, Reaktion des Aldehyds mit p-Toluensulfonylhydrazin zum entsprechenden p-Toluensulfonylhydrazon und nachfolgende Umwandlung in die Diazoverbindung durch Behandlung mit Triethylamin).
Ausbeute: 16%.
- **axiales Isomer:**: ³¹P-NMR (DMSO-d₆) : δ -4,62;
UV: λₘₐₓ (ε) : 322,5 (11500)
- **äquatoriales Isomer:**: ³¹P-NMR (DMSO-d₆) : δ -3, 18;
UV: λₘₐₓ (ε) : 321,5 (11000)

### Beispiel 3

### [7-(Carboxymethoxy)cumarin-4-yl]methylester von cGMP

Analog Beispiel 1 aus 1 mmol 8-Br-cGMP und 1,5 mmol [7-(tert.-butoxycarbonylmethoxy)cumarin-4-yl]diazomethan (hergestellt aus käuflichem 7-Hydroxy-4-methylcumarin durch Alkylierung mit Bromessigsäure-tert.-butylester, anschliessende Überführung in den (7-tert.-Butoxycarbonylmethoxy)-cumarin 4-carbaldehyd durch Oxidation mit Selendioxid, Reaktion des Aldehyds mit p-Toluensulfonylhydrazin zum entsprechenden p-Toluensulfonylhydrazon und nachfolgende Umwandlung in die Diazoverbindung durch Behandlung mit Triethylamin).
Ausbeute: 15%.
- **axiales Isomer:**: ³¹P-NMR (DMSO-d₆): δ -5,08;
UV: λₘₐₓ (ε): 326 (11500)
- **äquatoriales Isomer:**: ³¹P-NMR (DMSO-d₆): δ -4,07;
UV: λₘₐₓ (ε): 325 (11200)

### Beispiel 4

### [5,7-Bis(carboxymethoxy)cumarin-4-yl]methylester von cGMP

Analog Beispiel 1 aus 1 mmol cGMP und 1,5 mmol [5,7-Bis(tert.-butoxycarbonylmethoxy)cumarin-4-yl]diazomethan (hergestellt aus käuflichem 5,7-Dihydroxy-4-methylcumarin durch Alkylierung mit Bromessigsäure-tert.-butylester, anschließende Überführung in den 5,7-Bis(tert.-butoxycarbonylmethoxy)cumarin 4-carbaldehyd durch Oxidation mit Selendioxid, Reaktion des Aldehyds mit p-Toluensulfonylhydrazin zum entsprechenden p-Toluensulfonylhydrazon und nachfolgende Umwandlung in die Diazoverbindung durch Behandlung mit Triethylamin).
Ausbeute: 33%.
- **axiales Isomer:**: ³¹P-NMR (DMSO-d₆): δ -5,26;
UV: λₘₐₓ (ε): 324 (13100)
- **äquatoriales Isomer:**: ³¹P-NMR (DMSO-d₆): δ -4,17;
UV: λₘₐₓ (ε): 322 (13300)

### Beispiel 5

### [6,7-Bis(carboxymethoxy)cumarin-4-yl]methylester von 8-Br-cAMP

Analog Beispiel 1 aus 1 mmol 8-Br-cAMP und 1,5 mmol [6,7-Bis(tert.-butoxycarbonylmethoxy)cumarin-4-yl]diazomethan.
Ausbeute: 16%.
- **axiales Isomer:**: ³¹P-NMR (DMSO-d₆): δ -4,68;
UV: λₘₐₓ (ε): 347 (10300)
- **äquatoriales Isomer:**: ³¹P-NMR (DMSO-d₆): δ -2,64;
UV: λₘₐₓ (ε): 346 (10000)

### Beispiel 6

### [6,7-Bis(carboxymethoxy)cumarin-4-yl]methylester von 8-HrcGMP

Analog Beispiel 1 aus 1 mmol 8-Br-cGMP und 1,5 mmol [6,7-His(tert.-butoxycarbonylmethoxy)cumarin-4-yl]diazomethan.
Ausbeute: 25%.
- **axiales Isomer:**: ³¹P-NMR (DMSO-d₆): δ -4,96;
UV: λₘₐₓ (ε) : 349 (10200)
- **äquatoriales Isomer:**: ³¹P-NMR (DMSO-d₆) : δ -3,96;
UV: λₘₐₓ (ε) : 346 (12700)

### Beispiel 7

### [5,7-Bis(carboxymethoxy)cumaria-4-yl]methylester von 8-p-Chlorphenylthio-cAMP

Analog Beispiel 1 aus 1 mmol 8-pCPT-cAMP und 1,5 mmol [5,7-Bis(tert.-butoxycarbonylmethoxy)cumarin-4-yl]diazomethan.
Ausbeute: 30%.
- **axiales Isomer:**: UV: λₘₐₓ (ε) : 322 (13300)
- **äquatoriales Isomer:**: UV: λₘₐₓ (ε) : 321 (13300)

### Beispiel 8

### [6,7-Bis(methoxycarbonylmethoxy)cumarin-4-yl]methylester von cAMP

Analog Beispiel 1 aus 1 mmol cAMP und 1,5 mmol [6,7-Bis(methoxycarbonylmethoxy)cumarin-4-yl]diazomethan (hergestellt aus käuflichem 6,7-Dihydroxy-4-methylcumarin durch Alkylierung mit Bromessigsäuremethylester, anschliessende Überführung in den 6,7-Bis(methoxycarbonylmethoxy) cumarin 4-carbaldehyd durch Oxidation mit Selendioxid, Reaktion des Aldehyds mit p-Toluensulfonylhydrazin zum entsprechenden p-Toluensulfonylhydrazon und nachfolgende Umwandlung in die Diazoverbindung durch Behandlung mit Triethylamin) und Isolierung und Lyophilisierung der beiden Isomeren direkt nach der Isomerentrennung durch präparative HPLC.
Ausbeute: 15%.
- **axiales Isomer:**: ³¹P-NMR (DMSO-d₆): δ-4,57 ;
UV: λₘₐₓ (ε) : 340 (10700)
- **äquatoriales Isomer:**: ³¹P-NMR (DMSO-d₆) : δ -2,92;
UV: λₘₐₓ 339(ε) : 3 (10800)

### Beispiel 9

### [6,7-Bis(ethoxycarbonylmethoxy)cumaria-4-yl]methylester von cGMP

Analog Beispiel 7 aus 1 mmol cGMP und 1,5 mmol [6,7-Bis(ethoxycarbonylmethoxy)cumarin-4-yl]diazomethan (hergestellt aus käuflichem 6,7-Dihydroxy-4-methylcumarin durch Alkylierung mit Bromessigsäureethylester, anschließende Überführung in den 6,7-Bis(ethoxycarbonylmethoxy)cumarin 4-carbaldehyd durch Oxidation mit Selendioxid, Reaktion des Aldehyds mit p-Toluensulfonylhydrazin zum entsprechenden p-Toluensulfonylhydrazon und nachfolgende Umwandlung in die Diazoverbindung durch Behandlung mit Triethylamin).
Ausbeute: 25%.
- **axiales Isomer:**: ³¹P-NMR (DMSO-d₆) : δ-4,99 ;
UV: λₘₐₓ (ε) : 340 (11000)
- **äquatoriales Isomer :**: ³¹P-NMR (DMSO-d₆) : δ -3,79;
UV: λₘₐₓ 339(ε) : 3 (11000)

### Beispiel 10

Die nachstehend aufgeführten Verbindungen wurden wie in den vorhergehenden Beispielen beschrieben hergestellt.
[5,7-Bis(carboxymethoxy)cumarin-4-yl]methylester von cAMP
[7-(Carboxymethoxy)cumarin-4-yl]methylester von cAMP
[6,7-Bis(ethoxycarbonylmethoxy)cumarin-4-yl]methylester von cAMP
[6,7-Bis(carboxymethoxy)cumarin-4-yl]methylester von cGMP
[7,8-Bis(carboxymethoxy)cumarin-4-yl]methylester von cGMP
[6,7-Bis(methoxycarbonylmethoxy)cumarin-4-yl]methylester von cGMP
[7-(Carboxymethoxy)cumarin-4-yl]methylester von 8-Br-cAMP
[5,7-Bis(carboxymethoxy)cumarin-4-yl]methylester von 8-Br-cAMP
[7,8-Bis(carboxymethoxy)cumarin-4-yl]methylester von 8-Br-cAMP
[6,7-Bis(methoxycarbonylmethoxy)cumarin-4-yl]methylester von 8-Br-cAMP
[6,7-Bis(ethoxycarbonylmethoxy)cumarin-4-yl]methylester von 8-Br-cAMP
[7-(Carboxymethoxy)cumarin-4-yl]methylester von 8-Br-cGMP
[5,7-Bis(carboxymethoxy)cumarin-4-yl]methylester von 8-Br-cGMP
[7,8-Bis(carboxymethoxy)cumarin-4-yl]methylester von 8-Br-cGMP
[6,7-Bis(methoxycarbonylmethoxy)cumarin-4-yl]methylester von 8-Br-cGMP
[6,7-Bis(ethoxycarbonylmethoxy)cumarin-4-yl]methylester von 8-Br-cGMP
[7-(Carboxymethoxy)cumarin-4-yl]methylester von 8-p-Chlorphenylthio-cAMP
[6,7-Bis(carboxymethoxy)cumarin-4-yl]methylester von 8-p-Chlorphenylthio-cAMP
[7,8-Bis(carboxymethoxy)cumarin-4-yl]methylester von 8-p-Chlorphenylthio-cAMP
[6,7-Bis(methoxycarbonylmethoxy)cumarin-4-yl]methylester von 8-p-Chlorphenylthio-cAMP
[6,7-Bis(ethoxycarbonylmethoxy)cumarin-4-yl]methylester von 8-p-Chlorphenylthio-cAMP
[7-(Carboxymethoxy)cumarin-4-yl]methylester von 8-p-Chlorphenylthio-cGMP
[6,7-Bis(carboxymethoxy)cumarin-4-yl]methylester von 8-p-Chlorphenylthio-cGMP
[5,7-Bis(carboxymethoxy)cumarin-4-yl]methylester von 8-p-Chlorphenylthio-cGMP
[7,8-Bis(carboxymethoxy)cumarin-4-yl]methylester von 8- p-Chlorphenylthio-cGMP
[6,7-Bis(methoxycarbonylmethoxy)cumarin-4-yl]methylester von 8-p-Chlorphenylthio-cGMP
[6,7-Bis(ethoxycarbonylmethoxy)cumarin-4-yl]methylester von 8-p-Chlorphenylthio-cGMP

### Beispiel 11

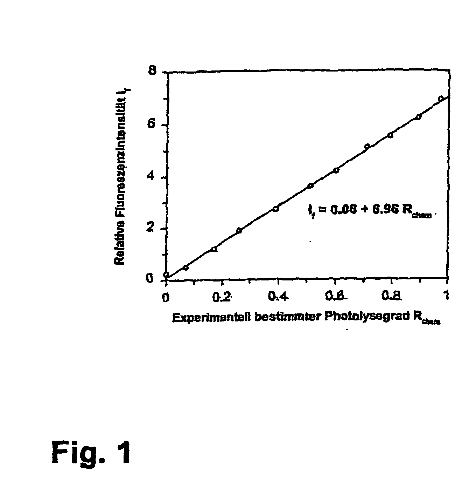

Die Abhängigkeit der Fluoreszenzintensität I_{f} vom Photolysegrad R_{chem} bei der Photolyse der axialen Form des erfindungsgemäßen [6,7-Bis (carboxymethoxy)-cumarin-4-yl] methylesters von cGMP in HEPES-Puffer (λₑₓ = 333 nm; λ_{f} = 420 nm) ist in Fig. 1 gezeigt.

### Beispiel 12

### Vergleich der Löslichkeiten verschiedener Cumarinylmethyl caged cyclischer Nucleotide in Acetonitril/HEPES-Puffer (5:95) bei pH 7,2 und Raumtemperatur

Eine Suspension der Verbindungen in dem aufgeführten Lösungsmittel wurde 5 Min. im Ultraschallbad behandelt, nichtgelöste Bestandteile wurden durch Zentrifugation abgetrennt und die Konzentration in der Lösung wurde mittels HPLC bestimmt. Folgende Sättigungskonzentrationen wurden ermittelt:

### Verbindungen des Standes der Technik:

(7-Methoxycumarin-4-yl)methyl-caged cAMP (axial): 15µM
(7-Methoxycumarin-4-yl)methyl-caged cGMP (axial): 15 µM
(7-Methoxycumarin-4-yl)methyl-caged cGMP (äquatorial): 25 µM
(7-Methoxycumarin-4-yl)methyl-caged 8-Br-cGMP (axial): 40 µM
(7-Methoxycumarin-4-yl)methyl-caged 8-Br-cGMP (äquatorial): 20µM

### Erfindungsgemäße Verbindungen:

[6,7-Bis(carboxymethoxy)-cumarin 4-yl]methyl-caged cGMP (axial): >500 µM
[6,7-Bis(carboxymethoxy)-cumarin 4-yl]methyl-caged cGMP (äquatorial): >1000 µM
[6,7-Bis(carboxymethoxy)-cumarin 4-yl]methyl-caged 8-Br-cGMP (axial): >500 µM
[6,7-Bis(carboxymethoxy)-cumarin 4-yl]methyl-caged 8-Br-cGMP (äquatorial): >1000 µM
[7-(Carboxymethoxy-cumarin 4-yl]methyl-caged cAMP (axial): 900 µM
[7-(Carboxymethoxy-cumarin 4-yl]methyl-caged cAMP (äquatorial): > 1000 µM

### Beispiel 13

### Aktivierung von cyclisch Nucleotid-abhängigen Ionen-Kanälen durch photolytische Freisetzung von cyclischen Nucleotiden aus entsprechenden caged Verbindungen

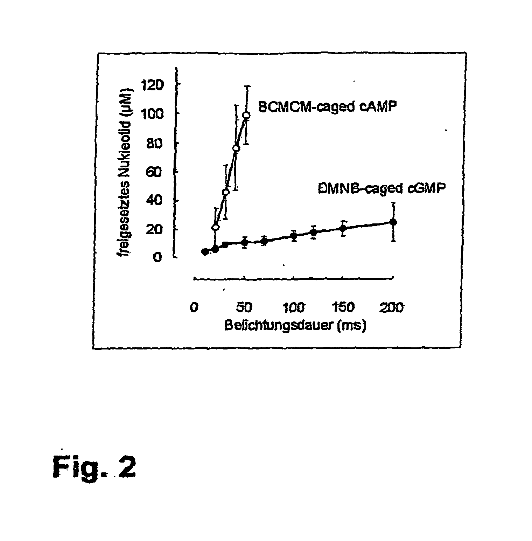

In Whole-Cell-Messungen an HEK293-Zellen wurde elektrophysiologisch die Aktivierung des cyclisch Nucleotid-abhängigen Ionenkanals CNCa3 (aus Riechzellen) durch photolytische Freisetzung von cAMP aus der axialen Form der Verbindung von Beispiel 1 (BCMCM-caged AMP) und des cyclisch Nucleotid-abhängigen Ionenkanals CNCα2 (aus Zapfen-Photorezeptoren) durch photolytische Freisetzung von cGMP aus der axialen Form des 4,5-Dimethoxy-2-nitrobenzylester von cGMP [DMNB-caged cGMP, hergestellt nach J. M. Nerbonne, S. Richard, J. Nargeot, H. A. Lester, Nature 310, 74 (1984)] vergleichend untersucht. Hierbei ergaben sich bei Einsatz einer intrazellulären Lösung von 500 µM der Verbindung von Beispiel 1 und einer intrazellulären Lösung von 200 µM 4,5-Dimethoxy-2-nitrobenzyl-caged cGMP und gleichartiger Belichtung mit UV-Licht (Lichtblitze 10 bis 200 ms, 100 W Hg-Kurzbogenlampe mit 335 nm cutoff-Filter) die in Fig. 2 verdeutlichten wesentlich höheren Konzentrationen an freigesetztem cyclischen Nucleotid für die Verbindung von Beispiel 1.

## Patentansprüche

1. Photolabile Cumarinylmethylester von cyclischen Nucleotiden der allgemeinen Formel I 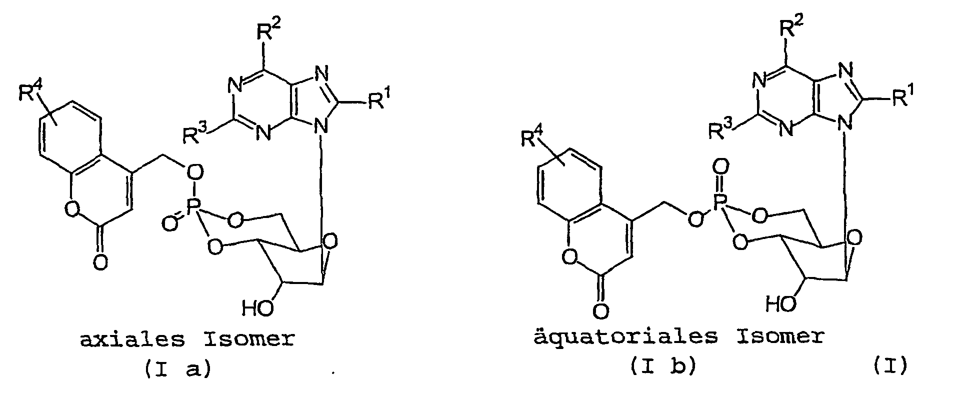 in der
R¹ Wasserstoff, Brom oder p-Chlorphenylthio bedeutet,
R⁴ 7-Carboxymethoxy, 6,7-, 5,7- oder 7,8- Bis(carboxymethoxy), 7-C₁-C₃-Alkoxycarbonylmethoxy, 6,7-Bis (C₁-C₃-alkoxycarbonylmethoxy), 5,7-Bis(C₁-C₃-alkoxycarbonylmethoxy) oder 7,8-Bis(C₁-C₃-alkoxycarbonylmethoxy) darstellt,
R² -NH₂ bedeutet und R³ Wasserstoff ist (Adeninrest) oder
R² -OH bedeutet und R³ -NH₂ darstellt (Guaninrest).

2. Verbindung der allgemeinen Formel I nach Anspruch 1,
**dadurch gekennzeichnet, daß**
R⁴ 7-Carboxymethoxy, 6,7-, 5,7- oder 7,8- His(carboxymethoxy) bedeutet.

3. Verbindungen der allgemeinen Formel I nach Anspruch 1,
**dadurch gekennzeichnet, daß**
R⁴ 6,7-Bis(carboxymethoxy) oder 6,7-Bis(C₁-C₃-alkoxycarbonylmethoxy) bedeutet.

4. Verbindungen der allgemeinen Formel I nach Anspruch 1,
**dadurch gekennzeichnet, daß**
C₁-C₃-Alkoxy im Rest R⁴ für den Methoxy- oder Ethoxyrest steht.

5. Verbindungen der allgemeinen Formel I nach einem der Ansprüche 1 bis 4 umfassend
1. [6,7-Bis(carboxymethoxy)cumarin-4-yl]methylester von cAMP
2. [7,8-Bis(carboxymethoxy)cumarin-4-yl]methylester von cAMP
3. [7-(Carboxymethoxy-cumarin-4-yl]methylester von cGMP
4. [5,7-Bis(carboxymethoxy)cumarin-4-yl]methylester von cGMP
5. [6,7-Bis(carboxymethoxy)cumarin-4-yl]methylester von 8-Br-cAMP
6. [6,7-His(carboxymethoxy)cumarin-4-yl]methylester von 8-Br-cGMP
7. [5,7-Bis(carboxymethoxy)cumarin-4-yl]methylester von 8-p-Chlorphenylthio-cAMP
8, [6,7-Bis(methoxycarbonylmethoxy)cumarin-4-yl]methylester von cAMP
9. 9.[6,7-Bis(ethoxycarbonylmethoxy)cumarin-4-yl]methylester von cGMP
10. {6,7-Bis(carboxymethoxy)cumarin-4-yl]methylester von cGMP
11. [7-(Carboxymethoxy-cumarin-4-yl]methylester von cAMP

6. Verbindungen nach einem der Ansprüche 1 bis 5 umfassend die äquatorialen Isomere I b.

7. Verfahren zur Herstellung von Cumarinylmethylestern von cyclischen Nucleotiden der allgemeinen Formel I gemäß Anspruch 1 mit R⁴ in der Bedeutung von 7- C₁-C₃-Alkoxycarbonylmethoxy, 6,7-Bis(C₁-C₃-alkoxycarbonylmethoxy), 5,7-Bis(C₁-C₃-alkoxycarbonylmethoxy) oder 7,8-Bis(C₁-C₃-alkoxycarbonylmethoxy),
**dadurch gekennzeichnet, daß**
cAMP, cGMP, 8-Brom-cAMP, 8-Brom-cGMP, 8-p-Chlorphenylthio-cAMP oder 8-p-Chlorphenylthio-cGMP mit C₁-C₃-alkoxycarbonylmethoxy-substituierten (Cumarin-4-yl)diazomethanen umgesetzt werden, das erhaltene Isomerengemisch gereinigt und gegebenenfalls in die Isomeren aufgetrennt wird.

8. Verfahren zur Herstellung von Cumarinylmethylestern von cyclischen Nucleotiden der allgemeinen Formel I gemäß Anspruch 1 mit R⁴ in der Bedeutung von 7-Carboxymethoxy, 6,7-, 5,7 oder 7,8- Bis(carboxymethoxy)
**dadurch gekennzeichnet, daß**
cAMP, cGMP, 8-Brom-cAMP, 8-Brom-cGMP, 8-p-Chlorphenylthio-cAMP oder 8-p-Chlorphenylthio-cGMP mit tert.butoxycarbonyl-methoxy-substituierten (Cumarin-4-yl)diazomethanen umgesetzt werden, die gebildeten tert.butoxycarbonyl-methoxy-substituierten Cumarinylmethylester gereinigt und gegebenenfalls in die Isomeren aufgetrennt werden und anschließend das Isomerengemisch oder die Isomeren mit Trifluoressigsäure behandelt werden.

9. Verfahren nach Anspruch 7 oder 8,
**dadurch gekennzeichnet, daß**
Reinigung und Isomerentrennung chromatographisch vorgenommen werden, vorzugsweise mittels flash-Chromatographie und/oder HPLC.

10. Verwendung von Verbindungen gemäß der Ansprüche 1 bis 6 zur Untersuchung cyclisch Nucleotid-abhängiger zellulärer Prozesse in biologischen in vitro Systemen.

11. Verwendung von Verbindungen gemäß der Ansprüche 1 bis 6 als biologisch inaktive photolabile Precursoren zur photochemischen in vitro Freisetzung von biologisch aktiven cAMP, cGMP, 8-Br-cAMP, 8-Br-cGMP, 8-p-Chlorphenylthio-cAMP oder 8-p-Chlorphenylthio-cGMP.

12. Verwendung nach Anspruch 10 oder 11,
**dadurch gekennzeichnet, daß**
die Verbindungen als Isomerengemische oder in Form ihrer reinen axialen oder reinen äquatorialen Isomeren eingesetzt werden.

## Claims

1. Photolabile coumarinylmethyl esters of cyclic nucleotides of general formula I 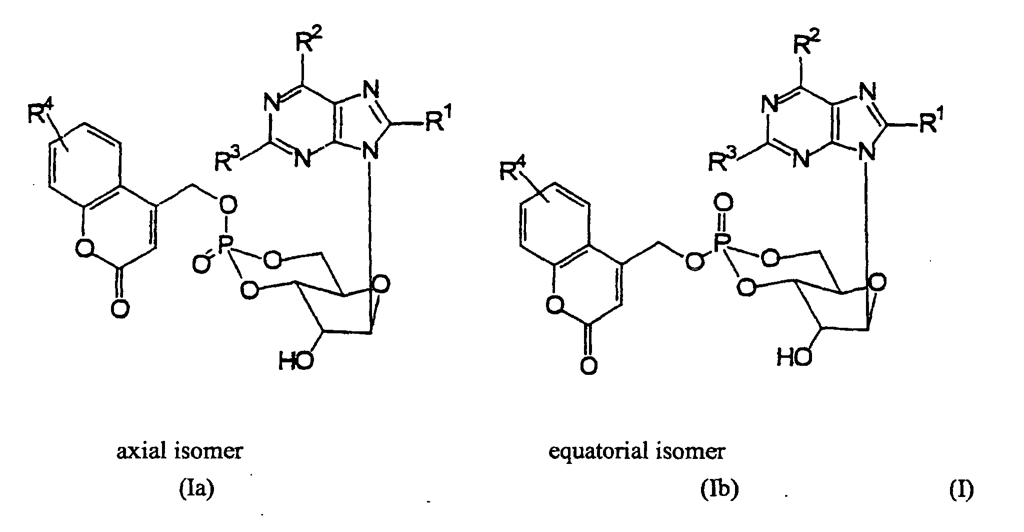 wherein
R¹ represents hydrogen, bromine or p-chlorophenylthio
R⁴ represents 7-carboxymethoxy, 6,7-, 5,7- or 7,8-bis(carboxymethoxy), 7-C₁-C₃-alkoxycarbonylmethoxy, 6,7-bis(C₁-C₃-alkoxycarbonylmethoxy), 5,7-bis(C₁-C₃-alkoxycarbonylmethoxy), or 7,8-bis(C₁-C₃-alkoxycarbonylmethoxy),
R² represents -NH₂ and R³ is hydrogen (adenine residue),
or
R² represents -OH and R³ represents -NH₂ (guanine residue).

2. The compounds of general formula I according to claim 1,
**characterized in that**
R⁴ represents 7-carboxymethoxy, 6,7-, 5,7- or 7,8-bis(carboxymethoxy).

3. The compounds of general formula I according to claim 1,
**characterized in that**
R⁴ represents 6,7-bis(carboxymethoxy) or 6,7-bis(C₁-C₃-alkoxycarbonylmethoxy).

4. The compounds of general formula I according to claim 1,
**characterized in that**
C₁-C₃-alkoxy in residue R⁴ represents a methoxy or ethoxy residue.

5. The compounds of general formula I according to any of claims 1 to 4, comprising
1. [6,7-bis-(carboxymethoxy)coumarin-4-yl]methyl ester of cAMP
2. [7,8-bis-(carboxymethoxy)coumarin-4-yl]methyl ester of cAMP
3. [7-(carboxymethoxycoumarin-4-yl]methyl ester of cGMP
4. [6,7-bis(carboxymethoxy)coumarin-4-yl]methyl ester of cGMP
5. [6,7-bis(carboxymethoxy)coumarin-4-yl]methyl ester of 8-Br-cAMP
6. [6,7-bis(carboxymethoxy)coumarin-4-yl]methyl ester of 8-Br-cGMP
7. [5,7-bis(carboxymethoxy)coumarin-4-yl]methyl ester of 8-p-chlorophenylthio-cAMP
8. [6,7-bis(methoxycarbonylmethoxy)coumarin-4-yl]methyl ester of cAMP
9. [6,7-bis(ethoxycarbonylmethoxy)coumarin-4-yl]methyl ester of cGMP
10. [6,7-bis(carboxymethoxy)coumarin-4-yl]methyl ester of cGMP
11. [7-(carboxymethoxy)coumarin-4-yl]methyl ester of cAMP.

6. The compounds according to any of claims 1 to 5, comprising the equatorial isomers Ib.

7. A method of producing the coumarinylmethyl esters of cyclic nucleotides of general formula I according to claim 1, wherein R⁴ represents 7-C₁-C₃-alkoxycarbonylmethoxy, 6,7-bis(C₁-C₃-alkoxycarbonylmethoxy), 5,7-bis(C₁-C₃-alkoxycarbonylmethoxy), or 7,8-bis(C₁-C₃-alkoxycarbonylmethoxy),
**characterized in that**
cAMP, cGMP, 8-bromo-cAMP, 8-bromo-cGMP, 8-p-chlorophenylthio-cAMP, or 8-p-chlorophenylthio-cGMP are reacted with C₁-C₃-alkoxycarbonylmethoxy-substituted (coumarin-4-yl)diazomethanes, the mixture of isomers obtained is purified and optionally separated to furnish the isomers.

8. A method of producing the coumarinylmethyl esters of cyclic nucleotides of general formula I according to claim 1, wherein R⁴ represents 7-carboxymethoxy, 6,7-, 5,7- or 7,8-bis(carboxymethoxy),
**characterized in that**
cAMP, cGMP, 8-bromo-cAMP, 8-bromo-cGMP, 8-p-chlorophenylthio-cAMP, or 8-p-chlorophenylthio-cGMP are reacted with tert-butoxycarbonylmethoxy-substituted (coumarin-4-yl)diazomethanes, the resulting tert-butoxycarbonylmethoxy-substituted coumarinylmethyl esters are purified and optionally separated to yield the isomers, and the mixture of isomers or the isomers are subsequently treated with trifluoroacetic acid.

9. The method according to claim 7 or 8,
**characterized in that**
purification and separation of the isomers are performed using chromatography, preferably flash chromatography and/or HPLC.

10. Use of compounds according to claims 1 to 6 for investigating cyclic nucleotide-dependent cellular processes in biological in vitro systems.

11. Use of compounds according to claims 1 to 6 as biologically inactive photolabile precursors in the photochemical in vitro liberation of biologically active cAMP, cGMP, 8-Br-cAMP, 8-Br-cGMP, 8-p-chlorophenylthio-cAMP, or 8-p-chlorophenylthio-cGMP.

12. The use according to claim 10 or 11,
**characterized in that**
the compounds are employed as mixtures of isomers or in the form of their pure axial or pure equatorial isomers.

## Revendications

1. Coumarinylméthyl esters photolabiles de nucléotides cycliques, de formule générale I 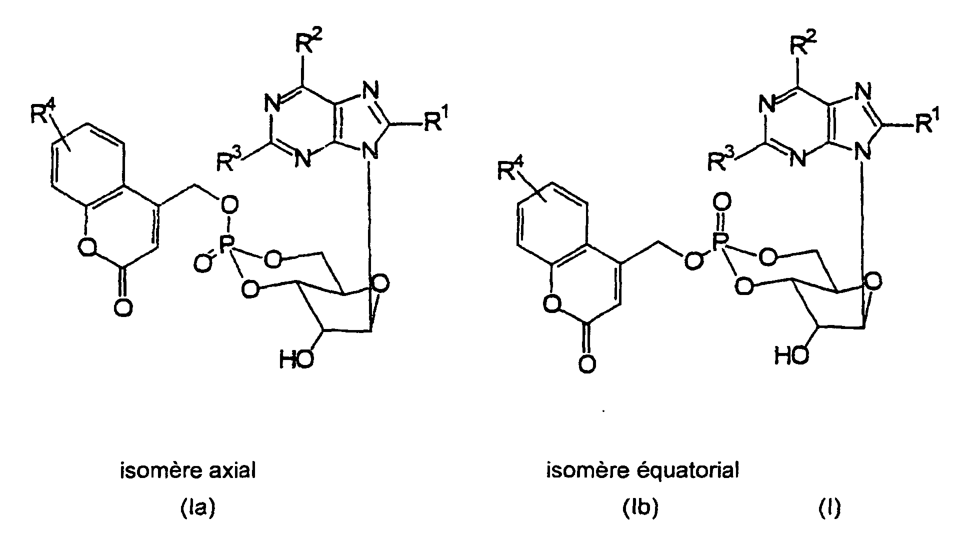 dans laquelle
R¹ représente un hydrogène, brome ou p-chlorophénylthio
R⁴ représente 7-carboxyméthoxy, 6,7-, 5,7- ou 7,8-bis(carboxyméthoxy), 7-C₁-C₃-alkoxycarbonylméthoxy, 6,7-bis(C₁-C₃-alkoxycarbonylméthoxy), 5,7-bis(C₁-C₃-alkoxycarbonylméthoxy), ou 7,8-bis(C₁-C₃-alkoxycarbonylméthoxy),
R² représente -NH₂ et R³ est un hydrogène (groupement d'adénine),
ou
R² représente -OH et R³ représente -NH₂ (groupement de guanine).

2. Composés de formule générale I selon la revendication 1,
**caractérisés en ce que**
R⁴ représente 7-carboxyméthoxy, 6,7-, 5,7- ou 7,8-bis(carboxyméthoxy).

3. Composés de formule générale I selon la revendication 1,
**caractérisés en ce que**
R⁴ représente 6,7-bis(carboxyméthoxy) ou 6,7-bis(C₁-C₃-alkoxycarbonylméthoxy).

4. Composés de formule générale I selon la revendication 1,
**caractérisés en ce que**
C₁-C₃-alkoxy dans le radical R⁴ représente un groupement méthoxy ou éthoxy.

5. Composés de formule générale I selon une des revendications 1 à 4, comprenant
1. [6,7-bis-(carboxyméthoxy)coumarin-4-yl]méthyl ester d'AMPc
2. [7,8-bis-(carboxyméthoxy)coumarin-4-yl]méthyl ester d'AMPc
3. [7-(carboxyméthoxycoumarin-4-yl]méthyl ester de GMPc
4. [5,7-bis(carboxyméthoxy)coumarin-4-yl]méthyl ester de GMPc
5. [6,7-bis(carboxyméthoxy)coumarin-4-yl]méthyl ester de 8-Br-AMPc
6. [6,7-bis(carboxyméthoxy)coumarin-4-yl]méthyl ester de 8-Br-GMPc
7. [5,7-bis(carboxyméthoxy)coumarin-4-yl]méthyl ester de 8-p-chlorophénylthio-AMPc
8. [6,7-bis(méthoxycarbonylméthoxy)coumarin-4-yl]méthyl ester d'AMPc
9. [6,7-bis(éthoxycarbonylméthoxy)coumarin-4-yl]méthyl ester de GMPc
10. [6,7-bis(carboxyméthoxy)coumarin-4-yl]méthyl ester de GMPc
11. [7-(carboxyméthoxy)coumarin-4-yl]méthyl ester d'AMPc.

6. Composés selon une des revendications 1 à 5, comprenant les isomères équatoriaux lb.

7. Procédé de préparation des coumarinylmethyl esters de nucléotides cycliques de formule générale I selon la revendication 1, dans laquelle R⁴ représente 7-C₁-C₃-alkoxycarbonylméthoxy, 6,7-bis(C₁-C₃-alkoxycarbonylméthoxy), 5,7-bis(C₁-C₃-alkoxycarbonylméthoxy), ou 7,8-bis(C₁-C₃-alkoxycarbonylméthoxy),
**caractérisé en ce**
**qu'**on fait réagir des AMPc, GMPc, 8-bromo-AMPc, 8-bromo-GMPc, 8-p-chlorophénylthio-AMPc, ou 8-p-chlorophénylthio-GMPc avec des (coumarin-4-yl)diazométhanes C₁-C₃-alkoxycarbonylméthoxy-substitués, on purifie le mélange d'isomères obtenu et on sépare éventuellement les isomères.

8. Procédé de préparation des coumarinylméthyl esters de nucléotides cycliques de formule générale I selon la revendication 1, dans laquelle R⁴ représente 7-carboxyméthoxy, 6,7-, 5,7- ou 7,8-bis(carboxyméthoxy),
**caractérisé en ce**
**qu'**on fait réagir des AMPc, GMPc, 8-bromo-AMPc, 8-bromo-GMPc, 8-p-chlorophénylthio-AMPc, ou 8-p-chlorophénylthio-GMPc avec des (coumarin-4-yl)diazométhanes tert-butoxycarbonylméthoxy-substitués, on purifie les coumarinylméthyl esters tert-butoxycarbonylméthoxy-substitués et on sépare éventuellement les isomères et on traite ensuite le mélange des isomères ou les isomères avec de l'acide trifluoroacétique.

9. Procédé selon la revendication 7 ou 8,
**caractérisé en ce**
**qu'**on effectue la purification et la séparation des isomères par chromatographie, de préférence par chromatographie en flash et/ou par HPLC.

10. Utilisation des composés selon les revendications 1 à 6 pour examiner les processus cellulaires dépendant des nucléotides cycliques dans les systèmes biologiques *in vitro.*

11. Utilisation des composés selon les revendications 1 à 6 en tant que précurseurs photolabiles biologiquement inactifs dans la libération photochimique *in vitro* des AMPc, GMPc, 8-Br-AMPc, 8-Br-GMPc, 8-p-chlorophénylthio-AMPc, ou 8-p-chlorophénylthio-GMPc biologiquement actifs.

12. Utilisation selon la revendication 10 ou 11,
**caractérisée en ce**
**qu'**on utilise les composés en tant que mélanges d'isomères ou sous forme de leurs isomères axiaux purs ou équatoriaux purs.
